# EUROPÄISCHE PATENTSCHRIFT

(11) **EP 2 451 374 B1**
(45) Veröffentlichungstag und Bekanntmachung des Hinweises auf die Patenterteilung: **04.09.2013**
(21) Anmeldenummer: 10724325.5
(22) Anmeldetag: 16.06.2010
(51) Int. Cl.: A61B 18/14, A61N 1/06, H01B 7/04, A61B 18/00, A61B 18/02

(54) **ELEKTROCHIRURGISCHES INSTRUMENT UND VERFAHREN ZUR HERSTELLUNG EINES ELEKTROCHIRURGISCHEN INSTRUMENTS**
ELECTROSURGICAL INSTRUMENT AND METHOD FOR PRODUCING AN ELECTROSURGICAL INSTRUMENT
INSTRUMENT ÉLECTRO-CHIRURGICAL, ET PROCÉDÉ DE PRODUCTION D'UN INSTRUMENT ÉLECTRO-CHIRURGICAL

(30) Priorität: 07.07.2009 DE 102009032065; 05.10.2009 DE 102009048312
(43) Veröffentlichungstag der Anmeldung: 16.05.2012
(60) Teilanmeldung: 13175509.2
(73) Patentinhaber: ERBE Elektromedizin GmbH, 72072 Tübingen (DE)
(72) Erfinder: SCHWAHN, Britta, 72074 Tübingen (DE); BESCH, Hansjörg, Björn, 72810 Gomaringen (DE)
(74) Vertreter: Rüger, Barthelt & Abel
(86) Internationale Anmeldenummer: PCT/EP2010/003595
(87) Internationale Veröffentlichungsnummer: WO 2011/003503

(56) Entgegenhaltungen:
- WO-A1-01/41664
- GB-A- 2 308 979
- US-A- 5 697 927
- US-A1- 2006 217 791
- US-A1- 2008 154 258

## Beschreibung

Die Erfindung betrifft ein elektrochirurgisches Instrument sowie ein Verfahren zur Herstellung eines elektrochirurgischen Instruments gemäß dem Anspruch 1 bzw. 8.

In der HF-Chirurgie (Hochfrequenzchirurgie) ist es bekannt, Gewebe gezielt durch die Applikation eines HF-Stroms zu devitalisieren. Ein entsprechender Vorgang kann durch ein monopolares oder bipolares Instrument vorgenommen werden. Bei der monopolaren Technik führt der Strompfad üblicherweise vom elektrochirurgischen Instrument des zu behandelnden Gewebes zur Neutralelektrode. Andererseits können bipolare Instrumente verwendet werden, die mit zwei voneinander elektrisch isolierten Abschnitten ausgebildet sind. Der Stromweg verläuft in diesem Fall von einem ersten Abschnitt des elektrochirurgischen Instruments über das zu behandelnde Gewebe zu einem zweiten Abschnitt des elektrochirurgischen Instruments.

Es sind Kryosonden bekannt, die Distal- und Proximalelektroden aufweisen, um einen entsprechenden Strom zu applizieren. Diese Kryosonden verfügen über eine Kühlvorrichtung, die es ermöglicht, das unmittelbar an dem Instrument anliegende Gewebe zu kühlen. Somit kann eine ungewollte Karbonisation des umliegenden Gewebes verhindert werden, so dass das Instrument zu einer kontrollierteren und möglicherweise grossflächigeren Devitalisierung eingesetzt werden kann. Des Weiteren lässt sich durch den gezielten Einsatz der Kühlvorrichtung die Wärmeverteilung entlang des Instruments steuern und somit der Devitalisierungsbereich einstellen.

Ein entsprechender Kühlvorgang kann beispielsweise durch das gezielte Einsetzen des Joule-Thomson-Effekts bewerkstelligt werden. Hierbei erfährt ein Fluid, insbesondere ein Gas, durch Drosselung (Druckänderung) eine Temperaturveränderung.

Es sind elektrochirurgische Instrumente bekannt, bei denen an der Außenseite eines Sondenschlauchs eine Distalelektrode und eine Proximalelektrode angeordnet sind. Im Lumen des Sondenschlauchs befindet sich ein Fluid- oder Gaskanal, der ein Kühfluid am distalen Ende des Sondenschlauchs in eine Expansionskammer bereitstellt. Das Lumen des Sondenschlauchs wird dazu verwendet, das expandierte Fluid abzuführen. Zur elektrischen Verbindung der Distalelektrode und der Proximalelektrode mit einem HF-Generator dienen zum einen der elektrisch leitfähige Gaskanal sowie eine in dem Lumen getrennt geführte und gegenüber dem Gaskanal isolierte Leitung. Die elektrische Verbindung der Proximalelektrode mit dieser Leitung gestaltet sich als schwierig. So werden Öffnungen in dem Sondenschlauch vorgesehen, um eine entsprechende Kontaktierung vorzunehmen. Durch diese Öffnungen kann das Fluid das im Lumen geführt wird, in das Organ eintreten und hier zu einer Schädigung führen. Der Fertigungsaufwand zur Herstellung derartiger Sonden, insbesondere entsprechender Bohrungen, ist hoch. Des Weiteren müssen die Sonden so dimensioniert werden, dass das Lumen zur Aufnahme der zusätzlichen Leitung ausreichend Platz bereitstellt. Für minimalinvasive Eingriffe ist es jedoch notwendig, dass der Durchmesser der Sonde sehr gering ist.

Aus der EP 1 902 683 A1 ist ein Katheter zur Applikation eines Stroms bekannt. Der Katheter umfasst hierfür mehrere Elektroden, die über Leiterbahnen innerhalb eines Schlauchs des Katheters mit einer geeigneten Spannung versorgt werden. Die Leiterbahnen befinden sich innerhalb der Außenwand des Schlauchs und sind durch diese elektrisch gegeneinander isoliert. Außerdem ist aus der US 2006/01217791 A1 ein Katheder mit einem schlauchartigen Grundkörper bekannt, der eine Anzahl von Lumen enthält, über die beispielsweise Fluide zu- oder abgeführt werden können. An der Außenseite des aus einem Kunststoff bestehenden schlauchartigen Grundkörpers sind zwei Leitungsfolien helixartig aufgebracht. Sie enden in einer oder mehreren Elektroden, die freiliegen, um mit einem medizinischen Instrument oder einem Nervenende in Berührung gebracht zu werden.

Ausgehend von diesem Stand der Technik ist es Aufgabe der vorliegenden Erfindung, ein elektrochirurgisches Gerät der eingangs genannten Art bereitzustellen, das sicher ist, geeignete Abmessungen aufweist und sich einfach und effizient herstellen lässt. Des Weiteren soll ein entsprechendes Verfahren angegeben werden.

Diese Aufgabe wird vorrichtungsseitig durch ein elektrochirurgisches Instrument gemäß dem Anspruch 1 und verfahrensseitig durch ein Verfahren gemäß dem Anspruch 8 gelöst.

Insbesondere wird die Aufgabe durch ein elektrochirurgisches Instrument gelöst, das einen Sondenschlauch zum Transport von Fluid, sowie eine Distalelektrode und eine Proximalelektrode, zur Devitalisierung von Gewebe umfasst. Das elektrochirurgische Instrument hat mindestens eine elektrische Leitung zum Bereitstellen einer HF-Spannung an der Elektrode, wobei die mindestens eine der elektrischen Leitungen eine Leitungsfolie umfasst, die entlang einer Längsachse des Sondenschlauchs angeordnet ist.

Ein besonderer Vorteil der vorliegenden Erfindung besteht also darin, dass eine elektrisch leitfähige Außenhaut, also die Leitungsfolie des Sondenschlauchs, als elektrische Leitung oder als Leiterbahn benutzt wird. Somit kann die elektrische Leitung für die Proximalelektrode bereitgestellt werden, ohne dass dies bei der Dimensionierung des Sondenschlauchs berücksichtigt werden müsste. Das Vorsehen der Leitungsfolie verursacht nur eine geringfügige Aufdickung der Wandstärke, so dass relativ kleine Sonden entwickelt werden können. Gegenüber dem bekannten Stand der Technik ist es nicht notwendig, den Sondenschlauch zu perforieren, um eine entsprechende Leitung herzustellen. Somit gestaltet sich auch der Herstellungsprozess eines erfindungsgemäßen elektrochirurgischen Instruments als relativ einfach. Das transportierte Fluid kann ein Gas, eine Flüssigkeit oder ein Gas-Flüssigkeits-Gemisch sein.

Die Leitungsfolie kann eine Kupferfolie, Goldfolie oder Silberfolie sein. Unter dem Begriff der Kupferfolie bzw. Goldfolie bzw. Silberfolie können sämtliche geeigneten Folien aus einer Kupferlegierung bzw. Goldlegierung bzw. Silberlegierung fallen. Durch das Verwenden der Kupferfolie ist es möglich, einen geeigneten Leiter mit geringer Wandstärke herzustellen. Kupfer hat auch einen niedrigen spezifischen Widerstand.

Die Leitungsfolie kann in einer schraubenförmigen Struktur entlang des Sondenschlauchs angeordnet sein. Diese helixartige Wicklung der Folie bewirkt, dass die nötige Flexibilität des Instruments, insbesondere des Sondenschlauchs, nur geringfügig beeinflusst wird. Dies sorgt dafür, dass das elektrochirurgische Instrument ausreichend navigationsfähig bleibt, so dass es problemlos innerhalb des Arbeitskanals eines Endoskops bewegt werden kann. Theoretisch wäre es zwar denkbar, die Leitungsfolien einseitig in Form einer direkten Leitungsbahn entlang der Längsrichtung des Sondenschlauchs zu führen oder eine zickzackförmige Folienbahn an einer Seite des Sondenschlauchs anzubringen, jedoch würde dies auch eine einseitige Änderung der Flexibilität und/oder Elastizität des Schlauches verursachen. Dies würde die Navigationsfähigkeit negativ beeinflussen.

Theoretisch könnten die einzelnen benachbarten Bahnen der Leitungsfolie zumindest abschnittsweise überlappend entlang der Längsrichtung des Sondenschlauchs angeordnet sein. Somit würde sich eine im Wesentlichen schlauchförmige Leitungsbahn ausbilden, die die Flexibilität des Sondenschlauchs nur geringfügig beeinflusst und nur einen geringen induktiven Widerstand aufweist. Vorzugsweise werden die benachbarten Bahnen der Leitungsfolie in Längsrichtung des Sondenschlauchs voneinander beabstandet angeordnet, so dass es zu keinen Überlappungen kommt. Die Überlappungen stellen nämlich eine zusätzliche Aufdickung bezüglich des Durchmessers des elektrochirurgischen Instruments dar. Der induktive Widerstand ist aufgrund der Breite der Bahnen der Leitungsfolie tolerierbar.

Das Instrument kann eine Isolatorschicht umfassen, die zur Fixierung der Leitungsfolie diese zumindest abschnittsweise überdeckt. Vorzugsweise ergibt sich also im Querschnitt eine Anordnung, die sich vom Inneren des Sondenschlauchs nach außen wie folgt gestaltet: Wand des Sondenschlauchs, Leitungsfolie, Isolatorschicht. Die Isolatorschicht kann sehr dünnwandig ausgestaltet sein und isoliert die durch die Leitungsfolie erzeugte Leiterbahn gegenüber dem Außenbereich. Des Weiteren schützt sie die Leitungsfolie vor äußeren Einflüssen und kann diese in Position halten. Die Isolatorschicht kann aus einem Material hergestellt werden, das die Flexibilität des Sondenschlauchs und somit des elektrochirurgischen Instruments nur geringfügig beeinflusst.

Die Proximalelektrode kontaktiert die Leitungsfolie unmittelbar. Somit kann die elektrische Verbindung zwischen der Leitungsfolie und der zugehörigen Elektrode denkbar einfach ausgebildet sein. Ein Löten ist an dieser Stelle nicht notwendig. Somit ist die Verbindungstechnik rein raumtauglich. Im Proximalbereich des Sondenschlauchs können entsprechende Anschlüsse auch durch eine mechanische Verbindungstechnik hergestellt werden. Beispielsweise kann die Kontaktierung durch ein Crimpen erfolgen.

Das elektrochirurgische Instrument ist eine Kryosonde mit einem Fluidkanal für ein Kühfluid (z.B. einem Gaskanal) sein, wobei der Fluidkanal ein elektrischer Leiter ist und zur Bereitstellung der HF-Spannung mit der Distalelektrode verbunden ist. Das elektrochirurgische Instrument ist also eine Kryosonde sein, die vorzugsweise eine in einem Lumen des Sondenschlauchs geführten Gaskanal umfasst. Das über den Fluidkanal eingebrachte Fluid kann aus einem Distalbereich der Sonde über das Lumen abgeführt werden. Erfindungsgemäß ist der Fluidkanal aus einem elektrisch leitfähigen Material hergestellt und dient somit ebenfalls als Leiterbahn. Diese Leiterbahn wird dazu genutzt, die Distalelektrode mit einem HF-Generator zu verbinden.

Die eingangs genannte Aufgabe wird ebenfalls durch ein Verfahren zur Herstellung eines elektrochirurgischen Instruments, insbesondere wie vorab beschrieben, gelöst, das die folgenden Schritte aufweist:
- Aufbringen einer Leitungsfolie auf einen Sondenschlauch, wobei sich die Leitungsfolie zumindest abschnittsweise entlang einer Längsachse des Sondenschlauchs erstreckt,
- Anordnen einer Proximalelektrode und/oder Distalelektrode derart an dem Sondenschlauch, dass die Proximalelektrode die Leitungsfolie abschnittsweise kontaktieren,
- Anordnen mindestens eines Anschlusses am Proximalende des Sondenschlauchs zur Verbindung der Leitungsfolie mit einem HF-Generator
- Anordnen eines Fluidkanals, vorzugsweise im Inneren des Sondenschlauchs, um in einem Distalbereich des ein Kühlfluid bereitzustellen, wobei der Fluidkanal ein elektrischer Leiter ist;
- Herstellen einer elektrischen Verbindung zwischen der Proximalelektrode und der Leitungsfolie sowie zwischen der Distalelektrode und dem Fluidkanal, um an der Proximalelektrode und der Distalelektrode eine HF-Spannung bereitzustellen.

Auch für dieses Verfahren ergeben sich ähnliche Vorteile wie für die bereits beschriebene Vorrichtung.

Weitere vorteilhafte Ausführungsformen ergeben sich anhand der Unteransprüche.

Nachfolgend wird die Erfindung anhand von einigen weiteren Ausführungsbeispielen beschrieben, die mittels Abbildungen näher erläutert werden. Hierbei zeigen:

Fig. 1 eine schematische Darstellung eines elektrochirurgischen Geräts mit Versorgungseinrichtung und Ablationssonde; und

Fig. 2 einen Schnitt durch die Ablationssonde nach Fig. 1.

In der nachfolgenden Beschreibung werden für gleiche und gleich wirkende Teile dieselben Bezugsziffern verwendet.

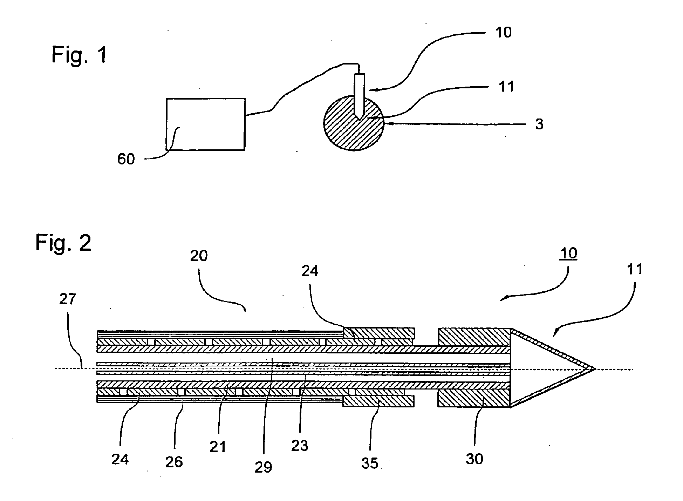

fig. 1 zeigt ein HF-Chirurgiegerät zur Devitalisierung von Gewebe. Eine Ablationssonde 10 ist über einen Versorgungsschlauch und mehrere Kabel mit einer Versorgungseinrichtung 60 verbunden. Die Ablationssonde 10 ist in ein Gewebe 3 eingeführt, das mittels eines HF-Stroms devitalisiert werden soll, der durch die Ablationssonde appliziert wird. Hierfür sind an der Ablationssonde 10 angeordnete Elektroden, nämlich eine Distalelektrode 30 und eine Proximalelektrode 35, mit einem HF-Generator in der Versorgungseinrichtung 60 verbunden. Um das Gewebe 3 vollständig zu devitalisieren, ist es notwendig, die Ablationssonde 10 zumindest abschnittsweise zu kühlen, um eine Karbonisation des die Ablationssonde 10 kontaktierenden Gewebes zu vermeiden. Daher umfasst die erfindungsgemäße Ablationssonde 10 eine Kühleinrichtung, die durch die Versorgungseinrichtung 60 gespeist wird.

Bei der Fig. 1 handelt es sich um eine schematische Darstellung, die die Wirkung der erfindungsgemäßen Ablationssonde 10 verdeutlichen soll. Tatsächlich werden derartige Sonden häufig über Endoskope an das Zielgewebe herangeführt, um einen minimalinvasiven Eingriff vorzunehmen. Hierfür ist es notwendig, den Durchmesser der Ablationssonde 10 so gering wie möglich zu halten. Des Weiteren muss die Ablationssonde 10 flexibel sein, um eine Devitalisierung auch an Orten vornehmen zu können, die nur schwer zugänglich sind. Bezüglich der Flexibilität sei noch angemerkt, dass Sonden, die sich in eine Richtung besser biegen lassen als in eine andere, von dem behandelnden Arzt häufig als ungeeignet empfunden werden, da es schwierig ist, derartige Ablationssonden 10 im Arbeitskanal zu führen.

Die erfindungsgemäße Ablationssonde 10 umfasst einen Sondenschlauch 21 (vgl. Fig. 2) mit einem proximalen und einem distalen Ende. Am distalen Ende des Sondenschlauchs 21 ist eine Sondenspitze 11 vorgesehen, die die Ablationssonde 10 abschließt. Am proximalen Ende des Sondenschlauchs 21 kann ein Handgriff zur Führung der Ablationssonde 10 und Anschlüsse zur Verbindung der Ablationssonde 10 mit der Versorgungseinrichtung 60 vorgesehen sein (in Fig. 1 nicht gezeigt).

Der Sondenschlauch 21 definiert eine theoretische Längsachse 27 der Ablationssonde 10, die sich vom proximalen zum distalen Ende der Ablationssonde 10 erstreckt.

Im Inneren des Sondenschlauchs 21, also in einem Lumen 29, befindet sich ein flexibler Gaskanal 23, der vom proximalen Ende der Ablationssonde 10 bis zur Sondenspitze 11 geführt ist. Über diesen Gaskanal 23 wird ein Fluid, vorzugsweise Lachgas, in die Ablationssonde 10 eingebracht. Das Fluid expandiert an einem distalen Ende des Gaskanals und entzieht der Ablationssonde 10 Wärmeenergie. Es kommt zu einer Abkühlung der Ablationssonde 10. Das expandierte Fluid wird über das Lumen 29 abgeführt und entsorgt. Somit sind der Sondenschlauch 21, der Gaskanal 23 und die Sondenspitze 11, die als Expansionskammer dient, ein Teil der Kühleinrichtung der Ablationssonde 10.

Am distalen Ende des Sondenschlauchs 21 in unmittelbarer Nachbarschaft zur Sondenspitze 11 befindet sich die Distalelektrode 30 , die den Sondenschlauch 21 ringförmig umschließt. Die Distalelektrode 30 steht in elektrischer Verbindung mit dem Gaskanal 23 , der aus einem elektrisch leitfähigen Material ausgebildet ist. Somit stellt der Gaskanal 23 zumindest einen Teil einer ersten Leiterbahn oder Leitung zur Versorgung der Distalelektrode 30 aus. Die Verbindung zwischen Distalelektrode 30 und Gaskanal 23 ist in der Fig. 2 nicht gezeigt. Beispielsweise kann die Distalelektrode 30 die vorzugsweise elektrisch leitfähige Sondenspitze 11 unmittelbar kontaktieren, die wiederum die Distalelektrode 30 kontaktiert oder ein Teil dieser ist.

Eine zweite Leiterbahn oder elektrische Leitung zur Versorgung der Proximalelektrode 35 wird durch eine Kupferfolie 24 ausgebildet. Auch bei der Proximalelektrode 35 handelt es sich um eine Ringelektrode, die den Sondenschlauch 21 umschließt und vorzugsweise koaxial zur Längsachse 27 angeordnet ist. Die Proximalelektrode 35 ist gegenüber der Distalelektrode 30 in proximaler Richtung versetzt und von der Distalelektrode 30 ausreichend beabstandet, um eine elektrische Isolationslücke bereitzustellen. Diese Lücke kann mit einem Isolator aufgefüllt werden.

Die Kupferfolie 24 ist unmittelbar auf den Sondenschlauch 21 aufgebracht und hat eine helixartige Wicklung, wobei sich die Kupferfolie 24 vom proximalen Ende des Sondenschlauchs bis unterhalb der Proximalelektrode 35 erstreckt. Die einzelnen Wicklungen um den Sondenschlauch 21 werden als Bahnen bezeichnet, wobei im gezeigten Ausführungsbeispiel die einzelnen Bahnen in Längsrichtungen einen im Wesentlichen gleichen Abstand aufweisen. Theoretisch wäre es möglich, die Abstände der einzelnen Bahnen zueinander zu variieren.

Die Helixstruktur oder helixartige Anordnung der Kupferfolie 24 hat eine konstante Steigung. Die Proximalelektrode 35 ist unmittelbar auf die Kupferfolie 24 aufgebracht und kontaktiert diese somit. Der elektrische Kontakt zwischen Kupferfolie 24 und Proximalelektrode 35 ist also sichergestellt.

Vorzugsweise umfasst die Ablationssonde 10 eine Außenisolierung 26, die auf die Kupferfolie 24 aufgebracht ist. Diese Außenisolierung 26 erstreckt sich vom proximalen Ende der Ablationssonde 10 bis zur Proximalelektrode 35. Die elektrische Kontaktierung der Kupferfolie 24 am proximalen Ende kann durch ein Crimpen erfolgen.

Der Sondenschlauch 21, die Kupferfolie 24, die Außenisolierung 26, die Distalelektrode 30 und die Proximalelektrode 35 bilden einen Sondenkörper 20.

Die Helixstruktur der Kupferfolie 24 ist ein Band, das sich mit konstanter Steigung entlang des Sondenschlauchs 21 windet. Die Wicklung, sowie die Außenisolierung 27 können derart ausgebildet sein, dass sie die Stabilität der Ablationssonde 10 erhöhen, wobei eine nötige Flexibilität erhalten bleibt.

Die beschriebene Kontaktierung zwischen Kupferfolie 24 und Proximalelektrode 35 lässt sich auch in Hinblick auf den Fertigungsprozess einfach herstellen. Diese vorgeschlagene elektrische Verbindung ist sicher. Da der Leiter oder die Leiterbahn an der Außenseite des Sondenschlauchs 21 geführt ist, müssen keine Durchführungen oder Bohrungen am Sondenschlauch 21 vorgesehen werden. Die Gasdichte des Sondenschlauchs 21 bleibt erhalten.

Im vorab beschriebenen Ausführungsbeispiel wurde ein bipolares elektrochirurgisches Instrument beschrieben, das mindestens zwei Elektroden aufweist. Für den Fachmann sollte es offensichtlich sein, dass die erfindungsgemäße Kontaktierung der Proximalelektrode 35 auch bei monopolaren Instrumenten sinnvoll eingesetzt werden kann. Auch sollte es klar sein, dass an Stelle der beschriebenen einen Kupferfolie 24 mehrere Kupferfolien parallel zueinander angeordnet werden können, um mehrere elektrisch voneinander isolierte Elektroden zu kontaktieren. Beispielsweise könnten die Distalelektrode 30 und die Proximalelektrode 35 mittels entsprechender Kupferfolien 24 versorgt werden. In diesem Fall könnte auf den elektrisch leitfähigen Gaskanal 23 verzichtet werden.

Im vorab beschriebenen Ausführungsbeispiel sind die Bahnen der Kupferfolie 24 parallel zueinander angeordnet. Es liegt keine Überlappung der Kupferfolie 24 vor.

Durch den engen Abstand der Wicklungen und die Breite der Kupferfolie 24 wirkt die Kupferfolie 24 fast wie ein Rohr und die entsprechenden induktiven Widerstände sind gering. Es wäre jedoch auch möglich, die Kupferfolie 24 überlappend anzuordnen, um den induktiven Widerstand zu verringern.

Die Ablationssonde 10 aus Fig. 2 weist zwei Ringelektroden auf, die den Sondenschlauch 21 umschließen. Erfindungsgemäß können jedoch auch beliebige andere Elektroden entlang des Sondenkörpers 20 der Ablationssonde 10 vorgesehen sein. Beispielsweise könnten die Ringelektroden abschnittsweise unterbrochen sein oder durch Plattenelektroden, die sich beispielsweise entlang der Längsachse 27 erstrecken, ersetzt werden.

Auch wenn vorhergehend eine Ablationssonde 10 beschrieben wurde, bei der ein Gas über einen Gaskanal 23 in den distalen Bereich der Ablationssonde 10 transportiert wird, um dieses dort zu expandieren, so ist die vorliegende Erfindung nicht auf diese spezielle Ausführungsform beschränkt. Die Erfindung lässt sich bei jeglicher Art von Schläuchen anwenden, wobei es irrelevant ist, ob diese zum Transport eines Gases, einer Flüssigkeit oder eines entsprechenden Gemisches verwendet werden.

### Bezugszeichenliste

3 : Gewebe
10 : Ablationssonde
11 : Sondenspitze
20 : Sondenkörper
21 : Sondenschlauch
23 : Gaskanal
24 : Kupferfolie
26 : Außenisolierung
27 : Längsachse
29 : Lumen
30 : Distalelektrode
35 : Proximalelektrode
60 : Versorgungseinrichtung

## Patentansprüche

1. Elektrochirurgisches Instrument, umfassend:
- einen Sondenschlauch (21) zum Transport von Fluid;
- eine Distalelektrode (30) und eine Proximalelektrode (35), zur Devitalisierung von Gewebe; und
- mindestens eine die Proximalelektrode (35) versorgende elektrische Leitung zum Bereitstellen einer HF-Spannung an der Elektrode (35),
wobei die Leitung eine Leitungsfolie (24) umfasst, die entlang einer Längsachse (27) des Sondenschlauchs (21) angeordnet ist, und
wobei das elektrochirurgische Instrument eine Kryosonde mit einem Fluidkanal (23) für ein Kühlfluid ist, wobei der Fluidkanal (23) ein elektrischer Leiter ist und zur Bereitstellung der HF-Spannung mit der Distalelektrode (30) verbunden ist oder diese kontaktiert.

2. Elektrochirurgisches Instrument nach Anspruch 1, **dadurch gekennzeichnet, dass** die Leitungsfolie (24) eine Gold-, Silber- oder Kupferfolie ist oder eine Legierung mit Gold- und/oder Silber- und/oder Kupfer-Komponenten umfasst.

3. Elektrochirurgisches Instrument nach einem der vorhergehenden Ansprüche, **dadurch gekennzeichnet, dass** die Leitungsfolie (24) in einer schraubenförmigen Struktur entlang des Sondenschlauchs (21) angeordnet ist.

4. Elektrochirurgisches Instrument nach Anspruch 3, **dadurch gekennzeichnet, dass** benachbarte Bahnen der schraubenförmigen Struktur in Längsrichtung des Sondenschlauchs (21) voneinander beabstandet sind.

5. Elektrochirurgisches Instrument nach einem der vorhergehenden Ansprüche, **gekennzeichnet durch** eine Isolatorschicht (26), die zur Fixierung der Leitungsfolie (24) diese zumindest abschnittsweise überdeckt.

6. Elektrochirurgisches Instrument nach einem der vorhergehenden Ansprüche, **dadurch gekennzeichnet, dass** die Proximalelektrode (35) die Leitungsfolie (24) unmittelbar kontaktiert.

7. Elektrochirurgisches Instrument nach einem der vorhergehenden Ansprüche, **dadurch gekennzeichnet, dass** der Fluidkanal (23) in einem Lumen (29) des Sondenschlauchs (21) angeordnet ist.

8. Verfahren zur Herstellung eines elektrochirurgischen Instruments, insbesondere nach einem der vorhergehenden Ansprüche, umfassend die Schritte:
- Aufbringen einer Leitungsfolie (24) auf einen Sondenschlauch (21), wobei sich die Leitungsfolie (24) zumindest abschnittsweise entlang einer Längsachse des Sondenschlauchs (21) erstreckt,
- Anordnen einer Proximalelektrode (35) und einer Distalelektrode (30) derart an dem Sondenschlauch (21), dass die Proximalelektrode (35) die Leitungsfolie abschnittsweise kontaktiert,
- Anordnen mindestens eines Anschlusses an einem Proximalende des Sondensctilauchs (21) zur Verbindung der Leitungsfolie (24) mit einem HF-Generator
- Anordnen eines Fluidkanals (23), vorzugsweise im Inneren des Sondenschlauchs, um in einem Distalbereich des Sondenschlauchs (21) ein Kühlfluid bereitzustellen, wobei der Fluidkanal (23) ein elektrischer Leiter ist;
- Herstellen einer elektrischen Verbindung zwischen der Proximalelektrode (35) und der Leitungsfolie (24) sowie zwischen der Distalelektrode (30) und dem Fluidkanal (23), um an der Proximalelektrode (35) und der Distalelektrode (30) eine HF-Spannung bereitzustellen.

9. Verfahren nach Anspruch 8, **dadurch gekennzeichnet, dass** die Leitungsfolie (24) eine Kupferfolie ist.

10. Verfahren nach Anspruch 8 oder 9, **dadurch gekennzeichnet, dass** die Leitungsfolie (24) in einer schraubenförmigen Struktur um den Sondenschlauch (21) entlang dessen Längsachse (27) angeordnet ist.

11. Verfahren nach Anspruch 10, **dadurch gekennzeichnet, dass** benachbarte Bahnen der schraubenförmigen Struktur in Längsrichtung des Sondenschlauchs (21) voneinander beabstandet angeordnet werden.

12. Verfahren nach einem der Ansprüche 8 bis 11, **gekennzeichnet durch** ein Anordnen einer isolatorschicht (26), die zur Fixierung der Leitungsfolie (24) diese zumindest abschnittsweise überdeckt.

## Claims

1. Electrosurgical instrument comprising:
• a probe tube (21) for transporting fluid;
• a distal electrode (30) and a proximal electrode (35) for devitalising tissue; and
• at least one electric lead supplying the proximal electrode (35) for provision of an HF voltage to the electrode (35),
wherein the lead comprises a conductive foil (24), which is arranged along a longitudinal axis (27) of the probe tube (21), and
wherein the electrosurgical instrument is a cryoprobe with a fluid duct (23) for a cooling fluid, wherein the fluid duct (23) is an electrical conductor and is connected to the distal electrode (30) for provision of the HF voltage or contacts this.

2. Electrosurgical instrument according to claim 1, **characterised in that** the conductive foil (24) is a gold, silver or copper foil or comprises an alloy with gold and/or silver and/or copper components.

3. Electrosurgical instrument according to one of the preceding claims, **characterised in that** the conductive foil (24) is arranged in a screw-shaped structure along the probe tube (21).

4. Electrosurgical instrument according to claim 3, **characterised in that** the adjacent paths of the screw-shaped structure are spaced from one another in the longitudinal direction of the probe tube (21).

5. Electrosurgical instrument according to one of the preceding claims, **characterised by** an insulator layer (26), which covers the conductive foil (24) at least in sections for fixture thereof.

6. Electrosurgical instrument according to one of the preceding claims, **characterised in that** the proximal electrode (35) contacts the conductive foil (24) directly.

7. Electrosurgical instrument according to one of the preceding claims, **characterised in that** the fluid duct (23) is arranged in a lumen (29) of the probe tube (21).

8. Method for the production of an electrosurgical instrument, in particular according to one of the preceding claims, comprising the steps:
• applying a conductive foil (24) to a probe tube (21), wherein the conductive foil (24) extends at least in sections along a longitudinal axis of the probe tube (21),
• arranging a proximal electrode (35) and a distal electrode (3) on the probe tube (21) in such a manner that the proximal electrode (35) contacts the conductive foil in sections,
• arranging at least one connection on a proximal end of the probe tube (21) to connect the conductive foil (24) to an HF generator,
• arranging a fluid duct (23), prefierably in the interior of the probe tube, to provide a cooling fluid in a distal region of the probe tube (21), wherein the fluid duct (23) is an electrical conductor;
• creating an electrical connection between the proximal electrode (35) and the conductive foil (24) and also between the distal electrode (30) and the fluid duct (23) in order to provide an HF voltage at the proximal electrode (35) and the distal electrode (30).

9. Method according to claim 8, **characterised in that** the conductive foil (24) is a copper foil.

10. Method according to claim 8 or 9, **characterised in that** the conductive foil (24) is arranged in a screw-shaped structure around the probe tube (21) along its longitudinal axis (27).

11. Method according to claim 10, **characterised in that** adjacent runs of the screw-shaped structure are spaced from one another in the longitudinal direction of the probe tube (21).

12. Method according to one of claims 8 to 11, **characterised by** arrangement of an insulator layer (26), which covers the conductive foil (24) at least in sections for fixture thereof.

## Revendications

1. Instrument électrochirurgical comprenant :
- un flexible de sonde (21) pour le transport de fluide;
- une électrode distale (30) et une électrode proximale (35) pour dévitaliser le tissu ; et
- au moins une ligne électrique qui alimente l'électrode proximale (35) et qui fournit à l'électrode (35) une tension HF,
la ligne comprenant un film conducteur (24) disposé suivant un axe longitudinal (27) du flexible de sonde (21) et
l'instrument électrochirurgical étant une cryosonde pourvue d'un canal de fluide (23) pour un fluide de refroidissement, le canal de fluide (23) étant un conducteur électrique et étant relié à l'électrode distale (30) ou contactant celle-ci pour fournir la tension HF.

2. Instrument électrochirurgical selon la revendication 1, **caractérisé en ce que** le film conducteur (24) est un film en or, en argent ou en cuivre ou comprend un alliage de composants à base d'or et/ou d'argent et/ou de cuivre.

3. Instrument électrochirurgical selon une des revendications précédentes, **caractérisé en ce que** le film conducteur (24) est disposé dans une structure hélicoïdale le long du flexible de sonde (21).

4. Instrument électrochirurgical selon la revendication 3, **caractérisé en ce que** des pistes voisines de la structure hélicoïdale sont disposées à distance les unes des autres dans la direction longitudinale du flexible de sonde (21).

5. Instrument électrochirurgical selon une des revendications précédentes, **caractérisé par** une couche isolante (26) qui, pour immobiliser le film conducteur (24), recouvre celui-ci au moins par endroits.

6. Instrument électrochirurgical selon une des revendications précédentes, **caractérisé en ce que** l'électrode proximale (35) contacte le film conducteur (24) directement.

7. Instrument électrochirurgical selon une des revendications précédentes, **caractérisé en ce que** le canal de fluide (23) est disposé dans une lumière (29) du flexible de sonde (21).

8. Procédé de fabrication d'un instrument électrochirurgical, en particulier selon une des revendications précédentes, comprenant les étapes consistant à :
- appliquer un film conducteur (24) sur un flexible de sonde (21), le film conducteur (24) s'étendant au moins par endroits suivant un axe longitudinal du flexible de sonde (21),
- disposer une électrode proximale (35) et une électrode distale (30) sur le flexible de sonde (21), de façon que l'électrode proximale (35) contacte le film conducteur par endroits,
- établir au moins une liaison avec une extrémité proximale du flexible de sonde (21) pour connecter le film conducteur (24) à un générateur HF
- disposer un canal de fluide (23), de préférence à l'intérieur du flexible de sonde, pour fournir un fluide de refroidissement dans une zone distale du flexible de sonde (21), le canal de fluide (23) étant un conducteur électrique ;
- établir une connexion électrique entre l'électrode proximale (35) et le film conducteur (24) ainsi qu'entre l'électrode distale (30) et le canal de fluide (23) afin de fournir une tension HF au niveau de l'électrode proximale (35) et de l'électrode distale (30).

9. Procédé selon la revendication 8, **caractérisé en ce que** le film conducteur (24) est un film de cuivre.

10. Procédé selon la revendication 8 ou 9, **caractérisé en ce que** le film conducteur (24) est disposé dans une structure hélicoïdale autour du flexible de sonde (21) suivant l'axe longitudinal (27) de celui-ci.

11. Procédé selon la revendication 10, **caractérisé en ce que** des pistes voisines de la structure hélicoïdale sont disposées à distance les unes des autres dans la direction longitudinale du flexible de sonde (21).

12. Procédé selon une des revendications 8 à 11, **caractérisé par** la présence d'une couche isolante (26) qui, pour immobiliser le film conducteur (24), recouvre celui-ci au moins par endroits.
